# FASCICULE DE BREVET EUROPEEN

(11) **EP 1 727 596 B1**
(45) Date de publication et mention de la délivrance du brevet: **09.09.2009**
(21) Numéro de dépôt: 05739582.4
(22) Date de dépôt: 16.03.2005
(51) Int. Cl.: A61K 8/84, A61K 8/88, A61Q 5/00

(54) **COMPOSITIONS COSMETIQUES COMPRENANT DES POLYAMINES MODIFIEES ET UTILISATIONS DESDITES COMPOSITIONS**
KOSMETISCHE ZUSAMMENSETZUNG MIT MODIFIZIERTEN POLYAMINEN UND IHRE VERWENDUNG
COSMETIC COMPOSITIONS CONTAINING MODIFIED POLYAMINES AND THE USE THEREOF

(30) Priorité: 17.03.2004 FR 0450532
(43) Date de publication de la demande: 06.12.2006
(73) Titulaire: L'ORÉAL, 75008 Paris (FR)
(72) Inventeur: LIVOREIL, Aude, F-75006 Paris (FR); VIC, Gabin, F-60280 Venette (FR)
(74) Mandataire: Catherine, Alain
(86) Numéro de dépôt international: PCT/FR2005/050169
(87) Numéro de publication internationale: WO 2005/092274

(56) Documents cités:
- EP-A- 0 112 593
- EP-A- 0 524 612
- DE-A- 10 226 416
- US-A- 5 756 080

## Description

La présente invention se rapporte à une composition cosmétique aqueuse comprenant une ou plusieurs polyamines modifiées particulières, ainsi que l'utilisation de certaines polyamines pour apporter de la douceur aux cheveux et pour améliorer l'homogénéité du dépôt d'un actif cosmétique sur les matières kératiniques et en particulier sur les cheveux.

L'utilisation de polyamines classiques, telles que par exemple les polyéthylèneimines, les polylysines, les polyornithines, est connue en cosmétique. Ces polyamines présentent en général une bonne affinité pour le cheveu en raison de leur caractère cationique.

Cependant, les propriétés cosmétiques de ces polyamines sont souvent insuffisantes. En particulier, elles présentent un caractère collant lorsqu'elle sont appliquées sur les fibres kératiniques, et leur dépôt sur la fibre est souvent irrégulier. Cela conduit à des touchers chargés à l'application et au cours des shampooings.

On connaît par ailleurs certaines polyamines modifiées, c'est-à-dire portant des groupes hydrophiles ou hydrophobes carbonés.

Ces polyamines modifiées peuvent notamment être utilisées en détergence ou pour apporter des qualités de conditionnement dans le textile.

Ainsi, la demande internationale WO 02/095122 divulgue l'utilisation de polyéthylèneimine et de polyvinylamines modifiées par des groupes hydrophobes contre le froissement des textiles.

La demande de brevet EP 0 112 593 et le brevet US 4,891,160 décrivent l'utilisation de polyamines éthoxylées dans des compositions détergentes.

Certaines polyamines modifiées sont également utilisées en cosmétique pour améliorer la qualité cosmétique des cheveux.

Ainsi, la demande internationale WO 02/15854 décrit l'utilisation de copolymères à greffons hydrophiles à base de vinylamine ou de vinylamide dans des compositions cosmétiques pour conférer un toucher agréable aux cheveux et un bon effet conditionnant.

Le brevet US 5,756,080 décrit l'utilisation de polyéthylèneimines siliconées pour conférer aux cheveux des propriétés conditionnantes améliorées, telle que la douceur par exemple.

La demande de brevet EP0524612 décrit l'utilisation d'un polydiméthylsiloxane modifié par un segment poly(N-acylalkylèneimine), pour conférer aux cheveux un aspect brillant, de la flexibilité et de la douceur.

La demande de brevet JP H8-217656 décrit l'utilisation de glycopolyaminoacides, en particulier un polyaminoacide sur lequel sont liés par liaison covalente des sucres et/ou leurs dérivés, pour la stimulation capillaire.

Le but de la présente invention est de fournir de nouvelles compositions contenant des polyamines modifiées par un ou plusieurs segments hydrocarbonés hydrophiles et/ou hydrophobes, ces polyamines modifiées permettant d'améliorer la qualité cosmétique des cheveux, notamment l'apport de douceur, de brillance et l'absence de toucher collant, et se répartissant de manière plus homogène sur les matières kératiniques et en particulier les cheveux, et permettant également d'améliorer l'homogénéité du dépôt d'un actif cosmétique sur les matières kératiniques.

L'invention a donc d'abord pour objet une composition cosmétique aqueuse comprenant, dans un milieu cosmétiquement acceptable, au moins un composé polymère dont la chaîne comprend au moins deux motifs aminés -NH- et/ou 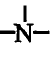 et ne contient pas d'unité vinylamine ou vinylamide, ledit composé polymère étant modifié par un ou plusieurs segments hydrocarbonés hydrophiles et/ou hydrophobes, le ou lesdits segments étant différents d'un sucre et ne contenant pas de groupement soufré, siliconé ou amidino, la modification par un segment hydrocarboné hydrophobe n'étant pas réalisée par l'intermédiaire d'un groupe espaceur bifonctionnel.

Par composé polymère modifié par un ou plusieurs segments hydrocarbonés hydrophiles et/ou hydrophobes, on entend au sens de la présente invention un composé polymère chimiquement lié à un ou plusieurs segments hydrocarbonés hydrophiles et/ou hydrophobes.

Par segment de sucre, on entend au sens de la présente invention tout segment de mono, oligo ou polysaccharide.

Par groupement amidino, on entend au sens de la présente invention, un groupement de formule :

-C(=NH)-NH₂

L'utilisation de ces composés polymères modifiés en cosmétique permet de combiner l'affinité importante des parties aminées pour le cheveu avec les propriétés cosmétiques apportées par les segments hydrophiles ou hydrophobes, telles que la douceur, la brillance, et l'absence de collant.

Les composés polymères utilisables dans la composition selon l'invention peuvent être linéaires, ramifiés, hyperbranchés ou dendrimériques.

Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications; une couche de chaînes terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèse connues, DP est compris entre 15 et 90%.

On peut également faire réagir un groupement terminal T sur le polymère hyperbranché pour obtenir une fonctionnalité particulière en extrémité de chaînes.

Plusieurs polymères hyperbranchés peuvent être associés entre eux, par une liaison covalente ou un autre type de liaison, par l'intermédiaire de leurs groupes terminaux. De tels polymères, dits pontés, entrent dans la définition des polymères hyperbranchés selon la présente invention.

Les dendrimères sont des polymères et oligomères hautement ramifiés ayant une structure chimique bien définie, et l'on dit que ce sont des polymères hyperbranchés « parfaits ».

En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la N^{ième} génération sont les groupes fonctionnels terminaux des fuseaux de la N^{ième} génération ou génération terminale.

La définition des dendrimères donnée ci-dessus inclut des molécules à ramifications symétriques. Elle inclut également des molécules à ramification non symétrique, comme par exemple les dendrimères dont les fuseaux sont des groupements lysine, dans lesquels le branchement d'une génération de fuseaux sur la précédente se fait sur les amines α et ε de la lysine, ce qui conduit à une différence dans la longueur des fuseaux des différentes ramifications.

Les polymères denses en étoiles, ou « dense star polymer », les polymères éclatés en étoile, ou « starburst polymer », les dendrimères en baguette, ou « rod-shaped dendrimer », sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de « dendrimères pontés », « agrégats de dendrimères » ou « bridged dendrimer ». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclue des ensembles monodisperses aussi bien que polydisperses de dendrimères.

Comme expliqué précédemment, la composition selon l'invention comprend au moins un composé polymère modifié par un ou plusieurs segments hydrophiles et/ou hydrophobes.

Dans la description qui suit, on décrit dans un premier temps les composés polymères utilisables, puis les segments hydrophiles et hydrophobes permettant de modifier lesdits composés polymères.

Les composés polymères compris dans la composition selon l'invention peuvent être choisis parmi :
- les polyalkylèneimines, de préférence les poly(alkylène (C₂-C₅) imines),
- les polyallylamines,
- les polymères contenant au moins 2 unités d'un ou plusieurs acides aminés basiques, choisis parmi l'ornithine, l'arspargine, la glutamine, la lysine et l'arginine.

Parmi les polyalkylèneimines utilisables dans la composition selon l'invention, on peut citer les polyéthylèneimines et les polypropylèneimines.

Les polyéthylèneimines (PEI) utilisables selon l'invention présentent généralement la formule suivante :

-(CH₂-CH₂-NH)ₙ-

où n est le nombre moyen d'unités éthylèneimine, n étant compris entre 5 et 10 000.

Les homopolymères d'éthylèneimine peuvent être branchés.

On peut citer en particulier la PEI-7 (n=7), la PEI-15 (n=15), la PEI-30 (n=30), la PEI-45 (n=45), la PEI-275 (n=275), la PEI-700 (n=700), la PEI-1000 (n=1000), la PEI-1400 (n=1400), la PEI-1500 (n=1500), la PEI-1750 (n=1750), et la PEI-2500 (n=2500).

Les polyéthylèneimines sont notamment décrites dans les documents : « KIRK-OTHMER ENCYCLOPEDIA OF CHEMICAL TECHNOLOGY», 3ème édition, vol.20, 1982, p.214-216, et « Polyethyleneimine Prospective Application » H.N. Feigenbaum, Cosmetics & Toiletries, 108, 1993, p.73.

Lesdits composés polymères contenant au moins 2 unités d'un ou plusieurs acides aminés basiques utilisables dans la composition selon l'invention contiennent généralement 2 à 10000 unités d'acide aminé basique.

Comme expliqué précédemment, les composés polymères utilisés dans la composition selon l'invention sont modifiés par un ou plusieurs segments hydrocarbonés hydrophiles et/ou hydrophobes, le ou lesdits segments étant différents d'un sucre et ne contenant pas de groupement soufré, siliconé ou amidino, la modification par un segment hydrocarboné hydrophobe n'étant pas réalisée par l'intermédiaire d'un groupe espaceur bifonctionnel.

Le ou les segments hydrophiles sont choisis parmi :
- les segments de composés polyhydroxylés, de préférence les segments de polyalkylèneglycol ou de polyvinylalcool,
- les segments de composés polycarboxylés comme par exemple les acides polyacryliques.

Les segments de polyalkylèneglycol sont généralement choisis parmi les segments de polyéthylèneglycol ou de polypropylèneglycol.

Par ailleurs, le ou les segments hydrophobes sont choisis 4e-parmi les chaînes grasses carbonées.

Les chaînes grasses carbonées sont choisies parmi les radicaux alkyle en C₁₀-C₅₀, les radicaux hydroxyalkyle en C₁₀-C₅₀, les radicaux carboxyalkyle en C₁₀-C₅₀, les radicaux ((C₁-C₁₀) alcoxy)carbonyl ((C₁₀-C₅₀) alkyl), les esters d'acides gras en C₁₂-C₅₀.

Ainsi, les composés polymères modifiés utilisables dans la composition selon l'invention peuvent être choisis parmi les polyéthylèneimine-polyéthylèneglycol, comme par exemple les composés commercialisés par la société BASF sous les références LUPASOL SC61 B et SC62J, ou par la société ALDRICH sous les références 42347-5 et 30618-5.

Les composés polymères modifiés utilisables dans la composition selon l'invention peuvent encore être choisis parmi les polyéthylèneimine-polyvinylalcool, les polyallylamine-polyéthylèneglycol et les polyallylamine-polyvinylalcool, notamment celles issues des polyallylamines commercialisées par la société NITTO BOSEKI Co.

Ils peuvent encore être choisis parmi les polylysine-polyéthylèneglycol et les polylysine-polyvinylalcool, en particulier les poly-ε-lysine-polyéthylèneglycol et les poly-ε-lysine-polyvinylalcool, issues par exemple de la poly-ε-lysine commercialisée par la société CHISSO.

Ils peuvent enfin être choisi parmi les polyéthylèneimines à chaînes grasses amidifiées par des acides gras, par exemple le composé commercialisé par la société BASF sous la référence LUPASOL ESA.

Les segments hydrophiles et/ou hydrophobes peuvent être greffés sur le ou les composés polymères. On obtient dans ce cas un polymère peigne. Ils peuvent également être séquencés avec les motifs aminés.

Une voie de synthèse de poly(acides aminés basiques) alcoxylés, notamment de polylysine-polyéthylèneglycol est décrite par exemple dans la demande internationale WO 00/71601.

Une voie de synthèse de polylysine polyéthylèneglycol est encore décrite dans la publication G.L. Kenausis et al, Journal of Physical Chemistry B, 2000, 104, p.3298.

Une voie de synthèse de polyéthylèneimine-polyéthylèneglycol est décrite par exemple dans la demande internationale WO 97/20879, ou encore dans la publication H. Petersen et al, Macromolecules, 2002, 35, p.6867.

Le ou les composés polymères modifiés représentent avantageusement de 0,01 à 40 %, de préférence de 0,1 à 20 %, mieux de 1 à 10 %, en poids du poids total de la composition.

Outre la présence d'un ou plusieurs composés polymères modifiés tels que décrits précédemment, la composition selon l'invention peut comprendre en outre avantageusement au moins un actif cosmétique choisi parmi les agents conditionneurs et les agents coiffants différents des polyamines de l'invention.

Les agents conditionneurs sont généralement choisis parmi les polymères cationiques, les silicones, volatiles ou non, linéaires ou cycliques, et les dérivés de silicones.

Par "polymère cationique", on entend tout polymère contenant des groupements cationiques et/ou des groupements ionisables en groupements cationiques.

Parmi les polymères cationiques, on peut citer plus particulièrement les polymères du type polyamine, polyaminoamide et polyammonium quaternaire.

Les polymères du type polyamine, polyaminoamide, polyammonium quaternaire, que l'on peut utiliser dans la composition de la présente invention, sont notamment décrits dans les brevets français FR 2 505 348 et FR 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français FR 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium ; on peut citer par exemple le polyquaternium 10 (nom INCI) ;
(4) les autres polysaccharides cationiques décrits plus particulièrement dans les brevets US 3 589 578 et US 4 031 307 tels que les gommes de guar contenant des groupements cationiques trialkylammonium ;
(5) les polymères constitués de motifs pipérazinyle et de groupes divalentes alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères. De tels polymères sont notamment décrits dans les brevets français FR 2 162 025 et FR 2 280 361 ;
(6) les polyaminoamides solubles dans l'eau, tels que ceux notamment décrits dans les brevets français FR 2 252 840 et FR 2 368 508 ;
(7) les dérivés de polyaminoamides, par exemple, les polymères acide adipique/dialkylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français FR 1 583 363 ;
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains US 3 227 615 et US 2 961 347 ;
(9) les cyclopolymères d'alkyldiallylamine ou de dialkyldiallylammo-nium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français FR 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020 ; on peut citer par exemple le chlorure d'hexadiméthrine (nom INCI), commercialisé par la société CHIMEX sous la référence MEXOMERE PO;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat^{®} FC 905, FC 550 et FC 370 par la société B.A.S.F;
(13) les polyamines comme le Polyquart^{®} H vendu par HENKEL, référencées sous le nom de " POLYETHYLENEGLYCOL (15) TALLOW POLYAMINE " dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE^{®} SC 92, SALCARE^{®} SC 95 et SALCARE^{®} SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi le chlorure d'hexadiméthrine et les homo ou copolymères de chlorure de diméthyldiallylammonium.

Comme expliqué précédemment, les agents conditionneurs peuvent également être choisis parmi les silicones.

Comme silicones utilisables comme actifs cosmétiques dans le procédé selon l'invention, on peut citer les polydiméthylsiloxanes, les polyorganosiloxanes quaternisés tels que ceux décrits dans la demande de brevet français FR 2 535 730, les polyorganosiloxanes à groupements aminoalkyles modifiés par des groupements alcoxycarbonylalkyles tels que ceux décrits dans le brevet US n° 4,749,732, des polyorganosiloxanes tels que le copolymère polydiméthylsiloxane-polyoxyalkyle du type diméthicone copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne décrits dans le brevet britannique FR 2 197 352, des polysiloxanes organo modifiés par des groupements mercapto ou mercaptoalkyles tels que ceux décrits dans le brevet français FR 1 530 369 et dans la demande de brevet européen EP 295 780.

Comme expliqué précédemment, outre les agents conditionneurs, la composition selon l'invention peut avantageusement contenir un ou plusieurs agents coiffants.

Les agents coiffants sont généralement choisis parmi les polymères amphotères, anioniques ou non ioniques.

Les polymères amphotères utilisables comme agents coiffants conformément à l'invention peuvent être choisis parmi les polymères comportant des motifs B et C répartis statistiquement dans une chaîne polymère, où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylates et acrylates, les dialkylaminoalkyl-méthacrylamides et acrylamides. De tels composés sont décrits dans le brevet américain n° 3 836 537. On peut également citer le copolymère acrylate de sodium/chlorure d'acrylamidopropyltriméthyl-ammonium vendu sous la dénomination POLYQUART^{®} KE 3033 par la Société HENKEL.
   Le composé vinylique peut être également un sel de dialkyldiallylammonium tel que le chlorure de diéthyldiallyl-ammonium.
   Les copolymères d'acide acrylique et de ce dernier monomère sont commercialisés sous les appellations MERQUAT^{®} 280, MERQUAT^{®} 295 et MERQUAT^{®} PLUS 3330 par la société CALGON.
(2) Les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quatemisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed, 1991) est Octylacrylamide/acrylates/butylaminoethyl-methacrylate copolymer tels que les produits vendus sous la dénomination AMPHOMER^{®} ou LOVOCRYL^{®} 47 par la société NATIONAL STARCH.
(3) Les polyaminoamides réticulés et alkylés partiellement ou totalement dérivant de polyaminoamides de formule générale : 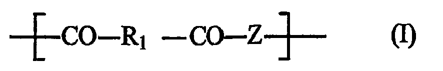 dans laquelle R₁ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 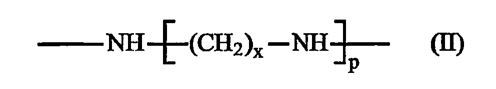 où x=2 et p=2 ou 3, ou bien x=3 et p=2 ce groupe dérivant de la diéthylène-triamine, de la triéthylène-tétraamine ou de la dipropylène-triamine;
   b) dans les proportions de 0 à 40 % en moles, le groupe (II) ci-dessus, dans lequel x=2 et p=1 et qui dérive de l'éthylènediamine, ou le groupe dérivant de la pipérazine 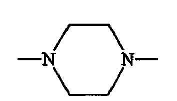
   c) dans les proportions de 0 à 20 % en moles, le groupe -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

   Les acides carboxyliques ou dicarboxyliques utilisables pour R₁ sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique.
   Les alcane-sultones utilisées dans l'alcoylation sont de préférence la propane- ou la butane-sultone. Les sels des agents d'alcoylation sont de préférence les sels de sodium ou de potassium.
(4) Les polymères comportant des motifs zwittérioniques de formule : 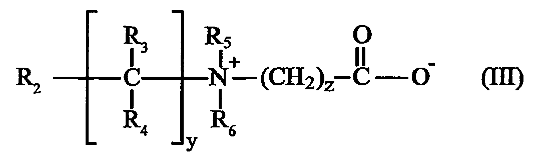 dans laquelle R₂ désigne un groupement insaturé polymérisable tel qu'un groupement acrylate, méthacrylate, acrylamide ou méthacrylamide, y et z représentent chacun un nombre entier de 1 à 3, R₃ et R₄ représentent un atome d'hydrogène, un groupement méthyle, éthyle ou propyle, R₅ et R₆ représentent un atome d'hydrogène ou un groupe alkyle de telle façon que la somme des atomes de carbone dans R₅ et R₆ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
   A titre d'exemple, on peut citer le copolymère de méthacrylate de méthyle et de diméthyl-carboxyméthylammonio-éthylméthacrylate de méthyle tel que le produit vendu sous la dénomination DIAFORMER^{®} Z301 par la société SANDOZ.
(5) Les polymères dérivés du chitosane comportant des motifs monomères répondant aux formules suivantes: 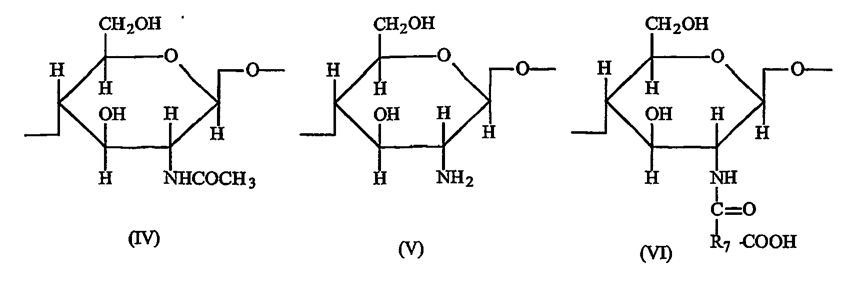 le motif (IV) étant présent dans des proportions comprises entre 0 et 30 %, le motif (V) dans des proportions comprises entre 5 et 50 % et le motif (VI) dans des proportions comprises entre 30 et 90 %, étant entendu que dans ce motif (VI), R₇ représente un groupe de formule: 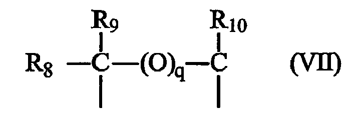 dans laquelle si q=0, R₈, R₉ et R₁₀, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₈, R₉ et R₁₀ étant dans ce cas un atome d'hydrogène ;
   ou si q=1, R₈, R₉ et R₁₀ représentent chacun un atome d'hydrogène, ainsi que les sels formés par ces composés avec des bases ou des acides.
(6) Les polymères dérivés de la N-carboxyalkylation du chitosane comme le N-carboxyméthyl-chitosane ou le N-carboxybutyl-chitosane vendu sous la dénomination EVALSAN^{®} par la société JAN DEKKER.
(7) Les polymères répondant à la formule générale (VIII) décrits par exemple, dans le brevet français FR 1 400 366: 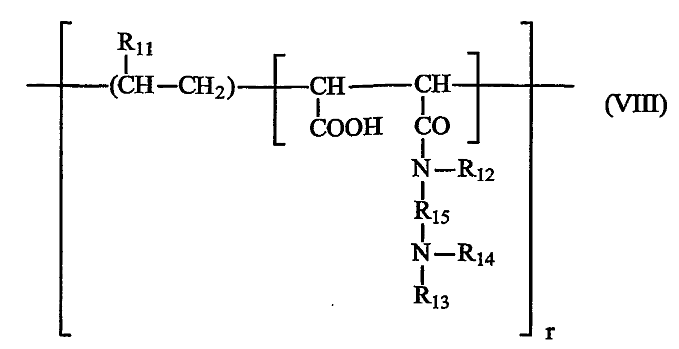 dans laquelle R₁₁ représente un atome d'hydrogène, un groupe CH₃O, CH₃CH₂O, phényle, R₁₂ désigne l'hydrogène ou un groupe alkyle inférieur tel que méthyle, éthyle, R₁₃ désigne l'hydrogène ou un groupe alkyle inférieur tel que méthyle, éthyle, R₁₄ désigne un groupe alkyle inférieur tel que méthyle, éthyle ou un groupe répondant à la formule: -R₁₅-N(R₁₃)₂, R₁₅ représentant un groupement -CH₂-CH₂-, -CH₂-CH₂-CH₂-, -CH₂-CH(CH₃)-, R₁₃ ayant les significations mentionnées ci-dessus,
   ainsi que les homologues supérieurs de ces groupes et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (IX)

      où D désigne un groupe 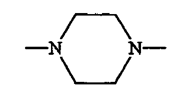 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques, les atomes d'oxygène, d'azote et de soufre étant présents sous forme de groupements éther, thioéther, sulfoxyde, sulfone, sulfonium, alkylamine, alcénylamine, hydroxyle, benzylamine, oxyde d'amine, ammonium quaternaire, amide, imide, alcool, ester et/ou uréthanne.
   b) Les polymères de formule :

      -D-X-D-X- (X)
   où D désigne un groupe 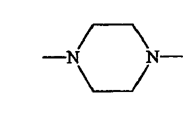 et X désigne le symbole E ou E" et au moins une fois E"; E ayant la signification indiquée ci-dessus et E" est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther/anhydride maléique modifié partiellement par semiamidification avec une N,N-dialkylaminoalkylamine telle que la N,N-diméthylaminopropyl-amine
ou par semiestérification avec une N,N-dialcanolamine. Ces copolymères peuvent également comporter d'autres comonomères vinyliques tels que le vinylcaprolactame.

Le ou les agents coiffants peuvent encore être choisis parmi les polymères anioniques.

Les polymères anioniques généralement utilisés dans la présente invention sont des polymères comportant des groupes dérivés d'acides carboxyliques, sulfoniques ou phosphoriques, et présentant une masse moléculaire en poids comprise entre 500 et 5 000 000.

Les groupements carboxyliques sont apportés par des monomères monoacides ou diacides carboxyliques insaturés tels que ceux répondant à la formule : 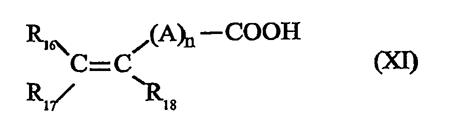 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₁₆ désigne un atome d'hydrogène, un groupement phényle ou benzyle, R₁₇ désigne un atome d'hydrogène, un groupement alkyle inférieur ou carboxyle, R₁₈ désigne un atome d'hydrogène, un groupement alkyle inférieur, un groupement -CH₂-COOH, phényle ou benzyle.

Dans la formule (XI) ci-dessus, le ou les groupements alkyles inférieurs comportent de préférence de 1 à 4 atomes de carbone et désignent, en particulier, les groupements méthyle et éthyle.

Les polymères anioniques à groupements carboxyliques préférés selon l'invention sont :
A) Les homo- ou copolymères d'acide acrylique ou méthacrylique ou leurs sels, et en particulier les produits vendus sous les dénominations VERSICOL^{®} E ou K par la société ALLIED COLLOID, ULTRAHOLD^{®} par la société BASF, les copolymères d'acide acrylique et d'acrylamide vendus sous la forme de leur sel de sodium sous les dénominations RETEN^{®} 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français FR 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER^{®} par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER^{®} MAEX par la société BASF.
C) Les copolymères dérivés d'acide crotonique tels que ceux comportant dans leur chaîne des motifs acétate ou propionate de vinyle, et éventuellement d'autres monomères tels que les esters allyliques ou méthallyliques, éthers vinyliques ou esters vinyliques d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros FR 1 222 944, FR 1 580 545, FR 2 265 782, FR 2 265 781, FR 1 564 110 et FR 2 439 798. Des produits commerciaux entrant dans cette classe sont par exemple les résines 28-29-30, 26-13-14 et 28-13-10 vendues par la société NATIONAL STARCH.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters. Ces polymères peuvent être estérifiés. De tels polymères sont décrits en particulier dans les brevets US 2 047 398, US 2 723 248, US 2 102 113, et dans le brevet GB 839 805. On peut citer notamment ceux vendus sous les dénominations GANTREZ^{®} AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français FR 2 350 384 et FR 2 357 241 de la demanderesse.
E) Les polyacrylamides comportant des groupements carboxylates.

Comme expliqué précédemment, les polymères anioniques peuvent être également des polymères comportant des groupes dérivés d'acides sulfoniques.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000 ainsi que les copolymères avec un comonomère insaturé tel que les acides acrylique
ou méthacrylique, et leurs esters, ainsi que l'acrylamide ou ses dérivés, les éthers vinyliques et la vinylpyrrolidone ;
- les sels de l'acide polystyrène-sulfonique, notamment les sels de sodium, ayant une masse moléculaire d'environ 500 000 et d'environ 100 000 vendus respectivement sous les dénominations Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER^{®} HSP 1180 par Henkel.

Enfin, les agents coiffants peuvent être choisis parmi les polymères non ioniques.

A titre de polymères non ioniques utilisables selon la présente invention, on peut notamment citer :
- les homopolymères de vinylpyrrolidone ;
- les copolymères de vinylpyrrolidone et d'acétate de vinyle ;
- les polyalkyloxazolines telles que les polyéthyloxazolines commer-cialisées par la société DOW CHEMICAL sous les dénominations PEOX^{®} 50 000, PEOX^{®} 200 000 et PEOX^{®} 500 000 ;
- les homopolymères d'acétate de vinyle tels que le produit proposé sous le nom de APPRETAN^{®} EM par la société HOECHST ou le produit proposé sous le nom de RHODOPAS^{®} A 012 par la société RHONE POULENC ;
- les copolymères d'acétate de vinyle et d'ester acrylique tels que le produit proposé sous le nom de RHODOPAS^{®} AD 310 de RHONE POULENC ;
- les copolymères d'acétate de vinyle et d'éthylène comme le produit proposé sous le nom de APPRETAN^{®} TV par la société HOECHST;
- les copolymères d'acétate de vinyle et d'ester maléique, par exemple de maléate de dibutyle tels que le produit proposé sous le nom de APPRETAN^{®} MB EXTRA par la société HOECHST ;
- les copolymères de polyéthylène et d'anhydride maléique ;
- les homopolymères d'acrylates d'alkyle et les homopolymères de méthacrylates d'alkyle tels que le produit commercialisé sous la dénomination MICROPEARL^{®} RQ 750 par la société MATSUMOTO ou le produit commercialisé sous la dénomination LUHYDRAN^{®} A 848 S par la société BASF ;
- les copolymères d'esters acryliques tels que, par exemple, les copolymères d'acrylates d'alkyle et de méthacrylates d'alkyle comme les produits commercialisés par la société ROHM & HAAS sous les dénominations PRIMAL^{®} AC -261 K et EUDRAGIT^{®} NE 30 D, par la société BASF sous les dénominations ACRONAL^{®} 601, LUHYDRAN^{®} LR 8833 ou 8845, par la société HOECHST sous les dénominations APPRETAN^{®} N 9213 ou N921 2 ;
- les copolymères d'acrylonitrile et d'un monomère non ionique choisi, par exemple, parmi le butadiène et les (méth)acrylates d'alkyle; on peut citer les produits proposés sous les dénominations NIPOL^{®} LX 531 8 par la société NIPPON ZEON ou ceux proposés sous la dénomination CJ 0601 8 par la société ROHM & HAAS ;
- les copolymères d'acétate d'alkyle et d'uréthanne tels que le produit 8538-33 par la société NATIONAL STARCH ;
- les polyamides tels que le produit ESTAPOR^{®} LO 11 proposé par la société RHONE POULENC ;
- les gommes de guar non ioniques chimiquement modifiées ou non modifiées.
   Les gommes de guar non ioniques non modifiées sont, par exemple, les produits vendus sous la dénomination VIDOGUM^{®} GH 175 par la société UNIPECTINE et sous la dénomination JAGUAR^{®} C par la société MEYHALL.
   Les gommes de guar non ioniques modifiées, utilisables selon l'invention, sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.
   Ces gommes de guar sont bien connues de l'état de la technique et peuvent, par exemple, être préparées en faisant réagir des oxydes d'alcènes correspondants, tels que, par exemple, des oxydes de propylène, avec la gomme de guar de façon à obtenir une gomme de guar modifiée par des groupements hydroxypropyle.
   De telles gommes de guar non-ioniques éventuellement modifiées par des groupements hydroxyalkyle sont, par exemple, vendues sous les dénominations commerciales JAGUAR^{®} HP8, JAGUAR^{®} HP60 et JAGUAR^{®} HP120, JAGUAR^{®} DC 293 et JAGUAR^{®} HP 105 par la société MEYHALL, ou sous la dénomination GALACTASOL^{®} 4H4FD2 par la société AQUALON.
   Les groupes alkyle des polymères non ioniques comportent de préférence de 1 à 6 atomes de carbone.
   Le ou les agents conditionneurs et/ou le ou les agents coiffants représentent généralement de 0,01 à 40 %, de préférence de 0,1 à 20 %, en poids par rapport au poids total de la composition selon l'invention.
   Outre les agents conditionneurs et les agents coiffants, la composition selon l'invention peut comprendre au moins un autre actif cosmétique choisi parmi les gélifiants et/ou épaississants minéraux ou organiques, associatifs ou non associatifs, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les agents bactéricides, et les agents anti-pelliculaires.
   Les constituants de la composition selon l'invention sont avantageusement compris dans un milieu cosmétiquement acceptable.
   Avantageusement, le milieu cosmétiquement acceptable est constitué d'au moins un solvant choisi parmi l'eau, les alcools en C₂-C₆, les éthers en C₂-C₆, les esters en C₂-C₆, la N-méthylpyrrolidone (NMP), les cétones en C₃-C₆, les polyols, et les éthers ou esters de polyols. La composition selon l'invention peut se présenter sous forme d'une lotion de soin capillaire, d'un shampooing ou d'un après-shampooing capillaire, d'un savon liquide ou solide de nettoyage du cuir chevelu, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire, de produits pour permanente
ou défrisage.

La composition selon l'invention peut être conditionnée dans un dispositif aérosol. Dans ce cas, elle comprend au moins un agent propulseur, qui peut être choisi parmi les hydrocarbures volatils, tels que le n-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

L'invention a encore pour objet l'utilisation pour apporter de la douceur aux cheveux, d'une composition cosmétique contenant, dans un milieu cosmétiquement acceptable, au moins un composé polymère dont la chaîne comprend au moins deux motifs aminés -NH- et/ou 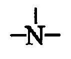 et ne contient pas d'unité vinylamine ou vinylamide, ledit composé polymère étant modifié par un ou plusieurs segments hydrocarbonés hydrophiles et/ou hydrophobes, le ou lesdits segments ne contenant pas de groupement soufré, siliconé ou amidino.

De préférence, le ou les segments hydrophiles ne sont pas des sucres.

L'invention a également pour objet l'utilisation en tant qu'actif cosmétique se répartissant de manière homogène sur les cheveux d'un composé polymère dont la chaîne comprend au moins deux motifs aminés
-NH- et/ou 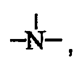 ledit composé polymère étant modifié par un ou plusieurs segments hydrocarbonés hydrophiles et/ou hydrophobes.

L'invention a enfin pour objet l'utilisation pour améliorer l'homogénéité du dépôt d'au moins un actif cosmétique sur les matières kératiniques, d'une composition cosmétique contenant, dans un milieu cosmétiquement acceptable, au moins un composé polymère dont la chaîne comprend au moins deux motifs aminés -NH- et/ou 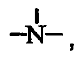 ledit composé polymère étant modifié par un ou plusieurs segments hydrocarbonés hydrophiles et/ou hydrophobes.

L'actif cosmétique dont l'homogénéité du dépôt est améliorée par le procédé selon l'invention peut être compris dans la composition ou être appliqué sur les matières kératiniques après l'application de la composition cosmétique.

Lorsque l'actif cosmétique est appliqué après l'application de la composition cosmétique, il est avantageusement de nature anionique.

De préférence, l'actif cosmétique dont l'homogénéité du dépôt est améliorée est choisi parmi les agents conditionneurs et les agents coiffants.

Les agents conditionneurs et les agents coiffants sont définis de la même manière que précédemment.

Ainsi, les agents conditionneurs peuvent être choisis parmi les polymères cationiques, les silicones, volatiles ou non, linéaires ou cycliques, et les dérivés de silicones.

De même, les agents coiffants peuvent être choisis parmi les polymères anioniques, non ioniques ou amphotères.

L'actif cosmétique peut également être choisi parmi tous les actifs cosmétiques classiques tels que les gélifiants et/ou épaississants minéraux ou organiques, associatifs ou non associatifs, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les agents bactéricides, et les agents anti-pelliculaires.

Les composés polymères utilisés selon l'invention peuvent être ceux définis précédemment au sujet de la composition selon l'invention.

Cependant, en outre, lorsque les composés polymères sont utilisés pour améliorer l'homogénéité d'un actif cosmétique sur les matières kératiniques selon l'invention, ils peuvent éventuellement comprendre une ou plusieurs unités vinylamine et/ou vinylamide.

Les segments hydrophiles et hydrophobes permettant de modifier les composés polymères utilisés selon l'invention peuvent être ceux définis précédemment au sujet de la composition selon l'invention.

Ainsi, les segments hydrophiles peuvent être choisis parmi :
- les segments de composés polyhydroxylés, de préférence les segments de polyalkylèneglycol ou de polyvinylalcool,
- les segments de composés polycarboxylés.

De même que précédemment, les segments de polyalkylèneglycol sont choisis de préférence parmi les segments de polyéthylèneglycol ou de polypropylèneglycol,

Par ailleurs, de même que précédemment, les segments hydrophobes sont choisis parmi les chaînes grasses carbonées.

Les chaînes grasses carbonées sont choisies de préférence parmi les alcools gras en C₁₂-C₅₀, les acides gras en C₁₂-C₅₀, les esters d'acides gras en C₁₂-C₅₀.

En outre, lorsque les composés polymères sont utilisés pour améliorer l'homogénéité d'un actif cosmétique sur les matières kératiniques selon l'invention, ils peuvent être modifiés par des segments de sucres ou par des segments siliconés.

Les sucres peuvent être choisis par exemple parmi les polysaccharides. On peut citer en particulier le glucose, le galactose, le maltose, le cellobiose et le maltotriose.

Les segments siliconés peuvent être par exemple des segments polydiméthylsiloxane.

Ainsi, les composés polymères modifiés utilisés pour améliorer l'homogénéité d'un actif cosmétique sur les matières kératiniques selon l'invention peuvent être choisis par exemple parmi les polyéthylèneimine-polysaccharides, les polyéthylèneimine-mono, di et trisaccharides, comme la polyéthylèneimine-glucose, la polyéthylèneimine-galactose, la polyéthylèneimine-maltose, la polyéthylèneimine-cellobiose, la polyéthylèneimine-maltotriose.

En outre, de même que précédemment, les composés polymères modifiés utilisés selon l'invention peuvent être choisis parmi les polyéthylèneimine-polyéthylèneglycol, les polyéthylèneimine-polyvinylalcool, les polyallylamine-polyéthylèneglycol, les polyallylamine-polyvinylalcool, les polylysine-polyéthylèneglycol et les polylysine-polyvinylalcool.

De même que précédemment, le ou les composés polymères modifiés représentent de 0,01 à 40 %, de préférence de 0,1 à 20 %, mieux de 1 à 10 %, en poids du poids total de la composition.

Lorsque la composition est utilisée pour apporter de la douceur aux cheveux selon l'invention, elle peut comprendre tout actif cosmétique classique.

La composition selon l'invention peut être appliquée en mode rincé ou non rincé.

Après l'application de la composition selon l'invention, les cheveux peuvent subir une étape de chauffage, à une température de préférence supérieure à 30°C.

L'application peut précéder ou suivre tout traitement cosmétique classique. On peut citer en particulier la coloration, la mise en forme permanente des cheveux, les opérations de défrisage, de lissage, et de shampooing.

La présente invention est illustrée par les exemples suivants.

### Synthèse d'une polyéthylèneimine-polyéthylèneglycol

On prépare une solution aqueuse C d'une polyéthylèneimine à 5% en masse, en utilisant la polyéthylèneimine commercialisée par la société BASF sous la référence LUPASOL P.

On porte son pH à 8,5 par ajout d'une quantité nécessaire d'acide chlorhydrique 6N et de tampon borate.

Sous agitation et à température ambiante (environ 20°C), on ajoute dans la solution C 2,16g d'un composé polyéthèneglycol portant un ester activé, le o[(N-succinimidyl)succinyl]-o'-méthyl-polyéthylèneglycol, commercialisé par la société FLUKA sous la référence 85976.

L'agitation est maintenue pendant 6 heures.

On obtient un polymère de polyéthylèneimine- polyéthylèneglycol, qui est ensuite purifié par dialyse.

Ce polymère peut ensuite être utilisé selon l'invention.

### Exemple Application sur les cheveux d'un composé polymère modifié selon l'invention

On prépare les deux compositions suivantes :
Composition 1 : Composition contenant une polyéthylèneimine

| | |
|---|---|
| LUPASOL P (50% de matière active) | : 10 g |
| Eau | : qsp 100 g |

Composition 2: Composition contenant une polyéthylèneimine-polyéthylèneglycol

| | |
|---|---|
| ALDRICH 42347-5 | : 5 g |
| Eau | : qsp 100 g |

Les compositions 1 et 2 sont chacune appliquées sur des mèches différentes de cheveux propres.

Les mèches sont placées à 40°C pendant 30 minutes, puis rincées à l'eau.

### 1. Effet sur la douceur des cheveux

Les mèches traitées avec la composition 1 sont rigides et un toucher collant très chargé apparaît après 3 shampooings.

Après 10 shampooings, le dépôt de polymère est toujours présent mais le toucher des mèches est chargé et lourd.

Les mèches traitées avec la composition 2 ont un toucher glissant et légèrement fixé.

Après 3 shampooings, les mèches sont douces et enrobées. Après 10 shampooings, le dépôt de polymère est toujours présent et les mèches ont un toucher doux et lisse.

### 2. Effet sur l'homogénéité du dépôt d'un colorant

On teste l'effet de l'application des compositions 1 et 2 sur l'homogénéité du dépôt d'un colorant.

Après application des compositions 1 et 2 définies précédemment, chaque mèche est colorée par le colorant anionique Red 80.

Les mèches traitées au préalable par la composition 1, puis colorées au Red 80, présentent une coloration hétérogène, c'est-à-dire des zones colorées et des zones non colorées.

Les mèches traitées au préalable par la composition 2, puis colorées au Red 80, présentent une coloration uniforme.

## Revendications

1. Composition cosmétique aqueuse comprenant, dans un milieu cosmétiquement acceptable, au moins un composé polymère dont la chaîne comprend au moins deux motifs aminés -NH- et/ou 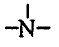 et ne contient pas d'unité vinylamine ou vinylamide, ledit composé polymère étant modifié par un ou plusieurs segments hydrocarbonés hydrophiles et/ou hydrophobes, le ou lesdits segments étant différents d'un sucre et ne contenant pas de groupement soufré, siliconé ou amidino, la modification par un segment hydrocarboné hydrophobe n'étant pas réalisée par l'intermédiaire d'un groupe espaceur bifonctionnel, le ou les composés polymères étant choisis parmi :
- les polyalkylèneimines, de préférence les poly(alkylène(C₂-C₅)imines),
- les polyallylamines,
- les polymères contenant au moins 2 unités d'un ou plusieurs acides aminés basiques choisis parmi l'ornithine, l'aspargine, la glutamine, la lysine et l'arginine, le ou les segments hydrophiles étant choisis parmi :
- les segments de composés polyhydroxylés, de préférence les segments de polyalkylèneglycol ou de polyvinylalcool,
- les segments de composés polycarboxylés, et le ou les segments hydrophobes étant des chaînes grasses carbonées choisies parmi les radicaux alkyle en C₁₀-C₅₀, les radicaux hydroxyalkyle en C₁₀-C₅₀, les radicaux carboxyalkyle en C₁₀-C₅₀, les radicaux ((C₁-C₁₀)alcoxy)carbonyl((C₁₀-C₅₀)alkyl), les esters d'acides gras en C₁₂-C₅₀.

2. Composition selon la revendication 1 **caractérisée en ce que** le ou les composés polymères sont linéaires, ramifiés, hyperbranchés ou dendrimériques.

3. Composition selon l'une quelconque des revendications précédentes **caractérisée en ce que** les segments de polyalkylèneglycol sont choisis parmi les segments de polyéthylèneglycol ou de polypropylèneglycol.

4. Composition selon l'une quelconque des revendications précédentes **caractérisée en ce que** le ou les segments hydrophobes et/ou hydrophiles sont greffés sur le ou les composés polymères ou séquencés avec les motifs aminés.

5. Composition selon l'une quelconque des revendications précédentes **caractérisée en ce que** le ou les composés polymères modifiés sont choisis parmi les polyéthylèneimine-polyéthylèneglycol, les polyéthylèneimine-polyvinylalcool, les polyallylamine-polyéthylèneglycol, les polyallylamine-polyvinylalcool, les polylysine-polyéthylèneglycol et les polylysine-polyvinylalcool.

6. Composition selon l'une quelconque des revendications précédentes **caractérisée en ce que** le ou les composés polymères modifiés représentent de 0,01 à 40 %, de préférence de 0,1 à 20 %, mieux de 1 à 10 %, en poids du poids total de la composition.

7. Composition selon l'une quelconque des revendications précédentes **caractérisée en ce qu'**elle comprend au moins un actif cosmétique choisi parmi les agents conditionneurs et les agents coiffants.

8. Composition selon la revendication 7 **caractérisée en ce que** le ou les agents conditionneurs sont choisis parmi les polymères cationiques, les silicones, volatiles ou non, linéaires ou cycliques, et les dérivés de silicones.

9. Composition selon la revendication 7 **caractérisée en ce que** le ou les agents coiffants sont choisis parmi les polymères anioniques, non ioniques ou amphotères.

10. Composition selon l'une quelconque des revendications 7 à 9 **caractérisée en ce que** le ou les agents conditionneurs et/ou le ou les agents coiffants représentent de 0,01 à 40 %, de préférence de 0,1 à 20 %, en poids par rapport au poids total de la composition.

11. Composition selon l'une quelconque des revendications précédentes **caractérisée en ce qu'**elle comprend en outre au moins un actif cosmétique choisi parmi les gélifiants et/ou épaississants minéraux ou organiques, associatifs ou non associatifs, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les agents bactéricides, et les agents anti-pelliculaires.

12. Composition selon l'une quelconque des revendications précédentes **caractérisée en ce qu'**elle comprend au moins un solvant choisi parmi l'eau, les alcools en C₂-C₆, les éthers en C₂-C₆, les esters en C₂-C₆, la N-méthylpyrrolidone (NMP), les cétones en C₃-C₆, les polyols, et les éthers ou esters de polyols.

13. Utilisation pour apporter de la douceur aux cheveux, d'une composition cosmétique contenant, dans un milieu cosmétiquement acceptable, au moins un composé polymère dont la chaîne comprend au moins deux motifs aminés -NH-et/ou 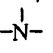 et ne contient pas d'unité vinylamine ou vinylamide, ledit composé polymère étant modifié par un ou plusieurs segments hydrocarbonés hydrophiles et/ou hydrophobes, le ou lesdits segments ne contenant pas de groupement soufré, siliconé ou amidino, le ou les composés polymères étant choisis parmi :
- les polyalkylèneimines, de préférence les poly(alkylène(C₂-C₅)imines),
- les polyallylamines,
- les polymères contenant au moins 2 unités d'un ou plusieurs acides aminés basiques choisis parmi l'ornithine, l'aspargine, la glutamine, la lysine et l'arginine, le ou les segments hydrophiles étant choisis parmi :
- les segments de composés polyhydroxylés, de préférence les segments de polyalkylèneglycol ou de polyvinylalcool,
- les segments de composés polycarboxylés, et le ou les segments hydrophobes étant des chaînes grasses carbonées choisies parmi les radicaux alkyle en C₁₀-C₅₀, les radicaux hydroxyalkyle en C₁₀-C₅₀, les radicaux carboxyalkyle en C₁₀-C₅₀, les radicaux ((C₁-C₁₀)alcoxy)carbonyl((C₁₀-C₅₀)alkyl), les esters d'acides gras en C₁₂-C₅₀.

14. Utilisation selon la revendication 13 **caractérisée en ce que** le ou les segments hydrophiles ne sont pas des sucres.

15. Utilisation pour améliorer l'homogénéité du dépôt d'au moins un actif cosmétique sur les matières kératiniques, d'une composition cosmétique contenant, dans un milieu cosmétiquement acceptable, au moins un composé polymère dont la chaîne comprend au moins deux motifs aminés -NH- et/ou 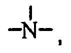 ledit composé polymère étant modifié par un ou plusieurs segments hydrocarbonés hydrophiles et/ou hydrophobes, le ou les composés polymères étant choisis parmi :
- les polyalkylèneimines, de préférence les poly(alkylène(C₂-C₅)imines),
- les polyallylamines,
- les polymères contenant au moins 2 unités d'un ou plusieurs acides aminés basiques choisis parmi l'ornithine, l'aspargine, la glutamine, la lysine et l'arginine, le ou les segments hydrophiles étant choisis parmi :
- les segments de composés polyhydroxylés, de préférence les segments de polyalkylèneglycol ou de polyvinylalcool,
- les segments de composés polycarboxylés, et le ou les segments hydrophobes étant des chaînes grasses carbonées choisies parmi les radicaux alkyle en C₁₀-C₅₀, les radicaux hydroxyalkyle en C₁₀-C₅₀, les radicaux carboxyalkyle en C₁₀-C₅₀, les radicaux ((C₁-C₁₀)alcoxy)carbonyl((C₁₀-C₅₀)alkyl), les esters d'acides gras en C₁₂-C₅₀.

16. Utilisation selon la revendication 15 **caractérisée en ce que** l'actif cosmétique est compris dans la composition cosmétique ou est appliqué sur les matières kératiniques après l'application de la composition.

17. Utilisation selon la revendication 15 ou 16 **caractérisée en ce que** l'actif cosmétique est choisi parmi les agents conditionneurs et les agents coiffants.

18. Utilisation selon la revendication 17 **caractérisée en ce que** les agents conditionneurs sont choisis parmi les polymères cationiques, les silicones, volatiles ou non, linéaires ou cycliques, et les dérivés de silicones.

19. Utilisation selon la revendication 17 **caractérisée en ce que** les agents coiffants sont choisis parmi les polymères anioniques, non ioniques ou amphotères.

20. Utilisation selon la revendication 15 ou 16 **caractérisée en ce que** l'actif cosmétique est choisi parmi les gélifiants et/ou épaississants minéraux ou organiques, associatifs ou non associatifs, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les agents bactéricides, et les agents anti-pelliculaires.

21. Utilisation selon l'une quelconque des revendications 13 à 20 **caractérisée en ce que** le ou les composés polymères sont linéaires, ramifiés, hyperbranchés ou dendrimériques.

22. Utilisation selon l'une quelconque des revendications 13 à 21 **caractérisée en ce que** les segments de polyalkylèneglycol sont choisis parmi les segments de polyéthylèneglycol ou de polypropylèneglycol.

23. Utilisation selon l'une quelconque des revendications 13 à 22 **caractérisée en ce que** les segments hydrophobes sont choisis parmi les chaînes grasses carbonées.

24. Utilisation selon la revendication 23 **caractérisée en ce que** les chaînes grasses carbonées sont choisies parmi les alcools gras en C₁₂-C₅₀ les acides gras en C₁₂-C₅₀, les esters d'acides gras en C₁₂-C₅₀.

25. Utilisation selon l'une quelconque des revendications 13 à 24 **caractérisée en ce que** le ou les segments hydrophobes et/ou hydrophiles sont greffés sur le ou les composés polymères ou séquencés avec les motifs aminés.

26. Utilisation selon l'une quelconque des revendications précédentes **caractérisée en ce que** le ou les composés polymères modifiés sont choisis parmi les polyéthylèneimine-polyéthylèneglycol, les polyéthylèneimine-polyvinylalcool, les polyallylamine-polyéthylèneglycol, les polyallylamine-polyvinylalcool, les polylysine-polyéthylèneglycol et les polylysine-polyvinylalcool.

## Claims

1. An aqueous cosmetic composition containing, in a cosmetically acceptable medium, at least one type of polymer compound whose chain comprises at least two types of amine units -NH- and/or 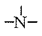 and is devoid of any vinyl amine or vinyl amide unit, said polymer compound being modified with one or more hydrophilic and/or hydrophobic hydrocarbon segments, said segment(s) being different from sugar and being devoid of any sulfur, silicone or amidino group, wherein the modification with hydrophobic hydrocarbon segments is not carried out by means of a bifunctional spacer group, the polymer compound(s) being selected from:
- polyalkyleneimines, preferably poly((C₂-C₅)alkyleneimines),
- polyallylamines,
- polymers containing at least 2 units of one or more basic amino acids selected from ornithin, asparagine, glutamine, lysine and arginine,
the hydrophilic segment(s) being selected from:
- segment(s) of polyhydroxylated compounds, preferably polyalkylene glycol or polyvinyl alcohol segments, and
- segments of polycarboxylated compounds, and
the hydrophobic segment(s) being selected from fatty acid carbon chains selected from C₁₀-C₅₀ alkyl radicals, C₁₀-C₅₀ hydroxyalkyl radicals, C₁₀-C₅₀ carboxyalkyl radicals, ((C₁-C₁₀) alkoxy)carbonyl ((C₁₀-C₅₀) alkyl) radicals, C₁₂-C₅₀ fatty acid esters.

2. A composition according to claim 1, **characterized in that** the polymer compound(s) is or are linear, branched, hyper-branched or dendrimeric.

3. A composition according to anyone of preceding claims, **characterized in that** the polyalkylene glycol segments are selected from polyethylene glycol or polypropylene glycol segments.

4. A composition according to anyone of preceding claims, **characterized in that** the hydrophobic and/or hydrophilic segment(s) is or are grafted onto the polymer compound(s) or sequenced with the amine units.

5. A composition according to anyone of preceding claims, **characterized in that** the modified polymer compound(s) is or are selected from polyethyleneimine-polyethylene glycol, polyethyleneimine-polyvinyl alcohol, polyallylamine-polyethylene glycol, polyallylamine-polyvinyl alcohol, polylysine-polyethylene glycol and polylysine-polyvinyl alcohol.

6. A composition according to anyone of preceding claims, **characterized in that** the modified polymer compound(s) represent(s) from 0.01 to 40%, preferably from 0.1 to 20%, more preferably from 1 to 10%, by weight as related to the composition total weight.

7. A composition according to anyone of preceding claims, **characterized in that** it comprises at least one cosmetic active agent selected from conditioning agents and styling agents.

8. A composition according to claim 7, **characterized in that** the conditioning agent(s) is or are selected from cationic polymers, volatile or non volatile, linear or cyclic silicones, and silicone derivatives.

9. A composition according to claim 7, **characterized in that** the styling agent(s) is or are selected from anionic, non ionic or amphoteric polymers.

10. A composition according to anyone of claims 7 to 9, **characterized in that** the conditioning agent(s) and/or the styling agent(s) represent from 0.01 to 40%, preferably from 0.1 to 20%, by weight as related to the composition total weight.

11. A composition according to anyone of preceding claims, **characterized in that** it comprises in addition at least one cosmetic active agent selected from gelling agents and/or inorganic or organic, associative or non associative thickening agents, anionic, non ionic, cationic or amphoteric surfactants, propenetrating agents, emulsifying agents, fragrances, preservatives, fillers, sunscreens, coloring agents, proteins, vitamins, provitamins, moisturizing agents, emollients, softening agents, mineral, vegetal or synthetic oils, hydrophilic or lipophilic active agents such as ceramides and pseudoceramides, antifoaming agents, antiperspirants, free radical scavengers, bactericides, and anti-dandruff agents.

12. A composition according to anyone of preceding claims, **characterized in that** it comprises at least one solvent selected from water, C₂-C₆ alcohols, C₂-C₆ ethers, C₂-C₆ esters, N-methylpyrrolidone (NMP), C₃-C₆ ketones, polyols, and polyol ethers or esters.

13. Use for providing softness to the hair, of a cosmetic composition that contains, in a cosmetically acceptable medium, at least one type of polymer compound whose chain comprises at least two types of amine units -NH- and/or 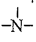 and is devoid of any vinyl amine or vinyl amide unit, said polymer compound being modified with one or more hydrophilic and/or hydrophobic hydrocarbon segments, said segment(s) being devoid of any sulfur, silicone or amidino group, the polymer compound(s) being selected from:
- polyalkyleneimines, preferably poly((C₂-C₅)alkyleneimines),
- polyallylamines,
- polymers containing at least 2 units of one or more basic amino acids selected from ornithin, asparagine, glutamine, lysine and arginine, the hydrophilic segment(s) being selected from:
- segment(s) of polyhydroxylated compounds, preferably polyalkylene glycol or polyvinyl alcohol segments, and
- segments of polycarboxylated compounds, and
the hydrophobic segment(s) being selected from fatty acid carbon chains selected from C₁₀-C₅₀ alkyl radicals, C₁₀-C₅₀ hydroxyalkyl radicals, C₁₀-C₅₀ carboxyalkyl radicals, ((C₁-C₁₀) alkoxy)carbonyl ((C₁₀-C₅₀) alkyl) radicals, C₁₂-C₅₀ fatty acid esters.

14. Use according to claim 13, **characterized in that** the hydrophilic segment(s) is or are not sugars.

15. Use for improving the deposition homogeneity of at least one cosmetic active agent onto the keratinic materials, of a cosmetic composition that contains, in a cosmetically acceptable medium, at least one type of polymer compound whose chain comprises at least two types of amine units -NH- and/or 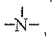 said polymer compound being modified with one or more hydrophilic and/or hydrophobic hydrocarbon segments, the polymer compound(s) being selected from:
- polyalkyleneimines, preferably poly((C₂-C₅)alkyleneimines),
- polyallylamines,
- polymers containing at least 2 units of one or more basic amino acids selected from ornithin, asparagine, glutamine, lysine and arginine,
the hydrophilic segment(s) being selected from:
- segment(s) of polyhydroxylated compounds, preferably polyalkylene glycol or polyvinyl alcohol segments, and
- segments of polycarboxylated compounds, and
the hydrophobic segment(s) being selected from fatty acid carbon chains selected from C₁₀-C₅₀ alkyl radicals, C₁₀-C₅₀ hydroxyalkyl radicals, C₁₀-C₅₀ carboxyalkyl radicals, ((C₁-C₁₀) alkoxy)carbonyl ((C₁₀-C₅₀) alkyl) radicals, C₁₂-C₅₀ fatty acid esters.

16. Use according to claim 15, **characterized in that** the cosmetic active agent is included in the cosmetic composition or is applied onto the keratinic materials once the composition has been applied.

17. Use according to claim 15 or 16, **characterized in that** the cosmetic active agent is selected from conditioning agents and styling agents.

18. Use according to claim 17, **characterized in that** conditioning agents are selected from cationic polymers, volatile or non volatile, linear or cyclic silicones, and silicone derivatives.

19. Use according to claim 17, **characterized in that** the styling agents are selected from anionic, non ionic or amphoteric polymers.

20. Use according to claim 15 or 16, **characterized in that** the cosmetic active agent is selected from gelling agents and/or inorganic or organic, associative or non associative thickening agents, anionic, non ionic, cationic or amphoteric surfactants, propenetrating agents, emulsifying agents, fragrances, preservatives, fillers, sunscreens, coloring agents, proteins, vitamins, provitamins, moisturizing agents, emollients, softening agents, mineral, vegetal or synthetic oils, hydrophilic or lipophilic active agents such as ceramides and pseudoceramides, antifoaming agents, antiperspirants, free radical scavengers, bactericides, and anti-dandruff agents.

21. Use according to anyone of claims 13 to 20, **characterized in that** the polymer compound(s) is or are linear, branched, hyper-branched or dendrimeric.

22. Use according to anyone of claims 13 to 21, **characterized in that** polyalkylene glycol segments are selected from polyethylene glycol or polypropylene glycol segments.

23. Use according to anyone of claims 13 to 22, **characterized in that** the hydrophobic segments are selected from fatty carbon chains.

24. Use according to claim 23, **characterized in that** fatty carbon chains are selected from C₁₂-C₅₀ fatty alcohols, C₁₂-C₅₀ fatty acids, C₁₂-C₅₀ fatty acid esters.

25. Use according to anyone of claims 13 to 24, **characterized in that** the hydrophobic and/or hydrophilic segments are grafted onto the polymer compound(s) or sequenced with the amine units.

26. Use according to anyone of preceding claims, **characterized in that** the modified polymer compound(s) is or are selected from polyethyleneimine-polyethylene glycol, polyethyleneimine-polyvinyl alcohol, polyallylamine-polyethylene glycol, polyallylamine-polyvinyl alcohol, polylysine-polyethylene glycol and polylysine-polyvinyl alcohol.

## Patentansprüche

1. Wässrige kosmetische Zusammensetzung, umfassend, in einem kosmetisch verträglichen Medium, wenigstens eine Polymerverbindung, deren Kette wenigstens zwei Aminmotive -NH- und/oder 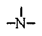 umfasst und keine Vinylamin- oder Vinylamid-Einheit enthält, wobei die Polymerverbindung durch ein oder mehrere hydrophile und/oder hydrophobe Kohlenwasserstoffsegmente modifiziert ist, wobei das Segment oder die Segmente von einem Zucker verschieden sind und keine schwefelhaltige, silikonisierte oder Amidino-Gruppe enthalten, wobei die Modifikation durch ein hydrophobes Kohlenwasserstoffsegment nicht über eine bifunktionelle Spacergruppe verwirklicht ist,
wobei die Polymerverbindung oder die Polymerverbindungen ausgewählt ist/sind aus:
- Polyalkyleniminen, vorzugsweise Poly(alkylen(C₂-C₅)iminen),
- Polyallylaminen,
- Polymeren, die wenigstens zwei Einheiten einer oder mehrerer basischer Aminosäuren, ausgewählt aus Ornithin, Asparagin, Glutamin, Lysin und Arginin, enthalten,
wobei das hydrophile Segment oder die hydrophilen Segmente ausgewählt ist/sind aus:
- Segmenten von Polyhydroxylverbindungen, vorzugsweise Polyalkylenglycol- und Polyvinylalkohol-Segmenten,
- Segmenten von polycarboxylierten Verbindungen, und das hydrophobe Segment oder die hydrophoben Segmente kohlenstoffhaltige Fettketten sind, ausgewählt aus (C₁₀-C₅₀)-Alkylresten, (C₁₀-C₅₀)-Hydroxyalkylresten, (C₁₀-C₅₀)-Carboxyalkylresten, ((C₁-C₁₀)Alkoxy)carbonyl((C₁₀-C₅₀)alkyl)-Resten, Estern von (C₁₂-C₅₀)-Fettsäuren.

2. Zusammensetzung gemäß Anspruch 1, **dadurch gekennzeichnet, dass** die Polymerverbindung oder die Polymerverbindungen linear, verzweigt, hyperverzweigt oder dendrimer ist/sind.

3. Zusammensetzung gemäß einem der vorangehenden Ansprüche, **dadurch gekennzeichnet, dass** die Polyalkylenglycol-Segmente ausgewählt sind aus Polyethylenglycol-oder Polypropylenglycol-Segmenten.

4. Zusammensetzung gemäß einem der vorangehenden Ansprüche, **dadurch gekennzeichnet, dass** das hydrophobe und/oder hydrophile Segment oder die hydrophoben und/oder hydrophilen Segmente auf die Polymerverbindung oder die Polymerverbindungen gepfropft ist/sind oder mit den Aminmotiven in Sequenz ist/sind.

5. Zusammensetzung gemäß einem der vorangehenden Ansprüche, **dadurch gekennzeichnet, dass** die modifizierte Polymerverbindung oder die modifizierten Polymerverbindungen ausgewählt ist/sind aus Polyethylenimin-Polyethylenglycol, Polyethylenimin-Polyvinylalkohol, Polyallylamin-Polyethylenglycol, Polyallylamin-Polyvinylalkohol, Polylysin-Polyethylenglycol und Polylysin-Polyvinylalkohol.

6. Zusammensetzung gemäß einem der vorangehenden Ansprüche, **dadurch gekennzeichnet, dass** die modifizierte Polymerverbindung oder die modifizierten Polymerverbindungen 0,01 bis 40 Gew.-%, vorzugsweise 0,1 bis 20 Gew.-%, am bevorzugtesten 1 bis 10 Gew.-%, bezogen auf das Gesamtgewicht der Zusammensetzung, darstellt/darstellen.

7. Zusammensetzung gemäß einem der vorangehenden Ansprüche, **dadurch gekennzeichnet, dass** sie wenigstens einen kosmetischen Wirkstoff, ausgewählt aus Konditionierungsmitteln und Frisiermitteln, umfasst.

8. Zusammensetzung gemäß Anspruch 7, **dadurch gekennzeichnet, dass** das oder die Konditionierungsmittel ausgewählt ist/sind aus kationischen Polymeren, flüchtigen oder nicht-flüchtigen, linearen oder cyclischen Silikonen und den Derivaten von Silikonen.

9. Zusammensetzung gemäß Anspruch 7, **dadurch gekennzeichnet, dass** das oder die Frisiermittel aus anionischen, nicht-ionischen oder amphoteren Polymeren ausgewählt ist/sind.

10. Zusammensetzung gemäß einem der Ansprüche 7 bis 9, **dadurch gekennzeichnet, dass** das oder die Konditionierungsmittel und/oder das oder die Frisiermittel 0,01 bis 40 Ges.-%, vorzugsweise 0,1 bis 20 Gew.-%, bezogen auf das Gesamtgewicht der Zusammensetzung, darstellen.

11. Zusammensetzung gemäß einem der vorangehenden Ansprüche, **dadurch gekennzeichnet, dass** sie außerdem wenigstens kosmetischen Wirkstoff umfasst, der ausgewählt ist aus Geliermitteln und/oder Verdickungsmitteln, mineralisch oder organisch, assoziativ oder nicht-assoziativ, anionischen, nicht-ionischen, kationischen oder amphoteren grenzflächenaktiven Substanzen, penetrationsfördernden Mitteln, Emulgatoren, Parfums, Konservierungsmitteln, Füllstoffen, Sonnenschutzmitteln, färbenden Materialien, Proteinen, Vitaminen, Provitaminen, feuchtigkeitsspendenden Mitteln, Emollientien, reizmildernden Mitteln, mineralischen, pflanzlichen oder synthetischen Ölen, hydrophilen oder lipophilen Wirkstoffen wie Ceramiden und Pseudoceramiden, Entschäumungsmitteln, Antiperspirantien, Mitteln gegen freie Radikale, bakteriziden Mitteln und Mitteln gegen Schuppen.

12. Zusammensetzung gemäß einem der vorangehenden Ansprüche, **dadurch gekennzeichnet, dass** sie wenigstens ein Lösungsmittel umfasst, das ausgewählt ist aus Wasser, (C₂-C₆)-Alkoholen, (C₂-C₆)-Ethern, (C₂-C₆)-Estern, N-Methylpyrrolidon (NMP), (C₃-C₆)-Ketonen, Polyolen und Ethern oder Estern von Polyolen.

13. Verwendung einer kosmetischen Zusammensetzung, um dem Haar Weichheit zu verleihen, wobei die Zusammensetzung in einem kosmetisch verträglichen Medium, enthält wenigstens eine Polymerverbindung, deren Kette wenigstens zwei Aminmotive
- NH- und/oder 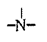 umfasst und keine Vinylamin- oder Vinylamid-Einheit enthält, wobei die Polymerverbindung durch ein oder mehrere hydrophile und/oder hydrophobe Kohlenwasserstoffsegment(e) modifiziert ist, wobei das Segment oder die Segmente keine schwefelhaltige, silikonisierte oder Amidino-Gruppe enthalten,
wobei die Polymerverbindung oder die Polymerverbindungen ausgewählt ist/sind aus:
- Polyalkyleniminen, vorzugsweise Poly(alkylen(C₂-C₅)iminen),
- Polyallylaminen,
- Polymeren, die wenigstens zwei Einheiten einer oder mehrerer basischer Aminosäuren, ausgewählt aus Ornithin, Asparagin, Glutamin, Lysin und Arginin, enthalten,
wobei das hydrophile Segment oder die hydrophilen Segmente ausgewählt ist/sind aus:
- Segmenten von Polyhydroxylverbindungen, vorzugsweise Polyalkylenglycol- und Polyvinylalkohol-Segmenten,
- Segmenten von polycarboxylierten Verbindungen, und das hydrophobe Segment oder die hydrophoben Segmente kohlenstoffhaltige Fettketten sind, ausgewählt aus (C₁₀-C₅₀)-Alkylresten, (C₁₀-C₅₀)-Hydroxyalkylresten, (C₁₀-C₅₀)-Carboxy-alkylresten, ((C₁-C₁₀)Alkoxy) carbonyl((C₁₀-C₅₀)alkyl)-Resten, Estern von (C₁₂-C₅₀)-Fettsäuren.

14. Verwendung gemäß Anspruch 13, **dadurch gekennzeichnet, dass** das hydrophile Segment oder die hydrophilen Segmente keine Zucker sind.

15. Verwendung zur Verbesserung der Homogenität der Abscheidung wenigstens eines kosmetischen Wirkstoffs einer kosmetischen Zusammensetzung auf Keratinmaterialien, wobei die kosmetische Zusammensetzung, in einem kosmetisch verträglichen Medium, wenigstens eine Polymerverbindung enthält, deren Kette wenigstens zwei Aminmotive -NH- und/oder 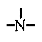 umfasst, wobei die Polymerverbindung durch ein oder mehrere hydrophile und/oder hydrophobe Kohlenwasserstoffsegmente modifiziert ist,
wobei die Polymerverbindung oder die Polymerverbindungen ausgewählt ist/sind aus:
- Polyalkyleniminen, vorzugsweise Poly(alkylen(C₂-C₅)iminen),
- Polyallylaminen,
- Polymeren, die wenigstens zwei Einheiten einer oder mehrerer basischer Aminosäuren, ausgewählt aus Ornithin, Asparagin, Glutamin, Lysin und Arginin, enthalten,
wobei das hydrophile Segment oder die hydrophilen Segmente ausgewählt ist/sind aus
- Segmenten von Polyhydroxylverbindungen, vorzugsweise Polyalkylenglycol- und Polyvinylalkohol-Segmenten,
- Segmenten von polycarboxylierten Verbindungen, und das hydrophobe Segment oder die hydrophoben Segmente kohlenstoffhaltige Fettketten sind, ausgewählt aus (C₁₀-C₅₀₎-Alkylresten, (C₁₀-C₅₀)-Hydroxyalkylresten, (C₁₀-C₅₀)-Carboxyalkylresten, ((C₁-C₁₀)Alkoxy)carbonyl((C₁₀-C₅₀)alkyl)-Resten, Estern von (C₁₂-C₅₀)-Fettsäuren.

16. Verwendung gemäß Anspruch 15, **dadurch gekennzeichnet, dass** der kosmetische Wirkstoff in der kosmetischen Zusammensetzung enthalten ist oder nach Auftragung der Zusammensetzung auf die Keratinmaterialien aufgetragen wird.

17. Verwendung gemäß Anspruch 15 oder 16, **dadurch gekennzeichnet, dass** der kosmetische Wirkstoff aus Konditionierungsmitteln und Frisiermitteln ausgewählt ist.

18. Verwendung gemäß Anspruch 17, **dadurch gekennzeichnet, dass** die Konditionierungsmittel ausgewählt sind aus kationischen Polymeren, flüchtigen oder nicht-flüchtigen, linearen oder cyclischen Silikonen und Derivaten von Silikonen.

19. Verwendung gemäß Anspruch 17, **dadurch gekennzeichnet, dass** die Frisiermittel aus anionischen, nicht-ionischen oder amphoteren Polymeren ausgewählt sind.

20. Verwendung gemäß Anspruch 15 oder 16, **dadurch gekennzeichnet, dass** der kosmetische Wirkstoff, ausgewählt ist aus Geliermitteln und/oder Verdickungsmitteln, mineralisch oder organisch, assoziativ oder nicht-assoziativ, anionischen, nicht-ionischen, kationischen oder amphoteren grenzflächenaktiven Substanzen, penetrationsfördernden Mitteln, Emulgatoren, Parfums, Konservierungsmitteln, Füllstoffen, Sonnenschutzmitteln, färbenden Materialien, Proteinen, Vitaminen, Provitaminen, feuchtigkeitsspendenden Mitteln, Emollientien, reizmildernden Mitteln, mineralischen, pflanzlichen oder synthetischen Ölen, hydrophilen oder lipophilen Wirkstoffen wie Ceramiden und Pseudoceramiden, Entschäumungsmitteln, Antiperspirantien, Mitteln gegen freie Radikale, bakteriziden Mitteln und Mitteln gegen Schuppen

21. Verwendung gemäß einem der Ansprüche 13 bis 20, **dadurch gekennzeichnet, dass** die Polymerverbindung oder die Polymerverbindungen linear, verzweigt, hyperverzweigt oder dendrimer ist/sind.

22. Verwendung gemäß einem der Ansprüche 13 bis 21, **dadurch gekennzeichnet, dass** die Polyalkylenglycol-Segmente ausgewählt sind aus Polyethylenglycol- oder Polypropylenglycol-Segmenten.

23. Verwendung gemäß einem der Ansprüche 13 bis 22, **dadurch gekennzeichnet, dass** die hydrophoben Segmente aus kohlenstoffhaltigen Fettketten ausgewählt sind.

24. Verwendung gemäß Anspruch 23, **dadurch gekennzeichnet, dass** die kohlenstoffhaltigen Fettketten ausgewählt sind aus (C₁₂-C₅₀)-Fettalkoholen, (C₁₂-C₅₀)-Fettsäuren, Estern von (C₁₂-C₅₀)-Fettsäuren.

25. Verwendung gemäß einem der Ansprüche 13 bis 24, **dadurch gekennzeichnet, dass** das hydrophobe und/oder hydrophile Segment oder die hydrophoben und/oder hydrophilen Segmente auf die Polymerverbindung oder die Polymerverbindungen gepfropft ist/sind oder mit den Aminmotiven in Sequenz ist/sind.

26. Verwendung gemäß einem der vorangehenden Ansprüche, **dadurch gekennzeichnet, dass** die modifizierte Polymerverbindung oder die modifizierten Polymerverbindungen ausgewählt ist/sind aus Polyethylenimin-Polyethylenglycol, Polyethylenimin-Polyvinylalkohol, Polyallylamin-Polyethylenglycol, Polyallylamin-Polyvinylalkohol, Polylysin-Polyethylenglycol und Polylysin-Polyvinylalkohol.
